# EUROPÄISCHE PATENTANMELDUNG

(11) **EP 1 930 031 A1**
(43) Veröffentlichungstag der Anmeldung: **11.06.2008**
(21) Anmeldenummer: 06023159.4
(22) Anmeldetag: 07.11.2006
(51) Int. Cl.: A61L 2/07, A61L 2/18

(54) **Verfahren zum Sterilisieren des Anschlussbereichs eines Bag-in-Tank Systems sowie eine entsprechende Anlage zum Sterilisieren**

(71) Anmelder: INDAG Gesellschaft für Industriebedarf mbH & Co. Betriebs KG, 69214 Eppelheim (DE)
(72) Erfinder: Wild, Hans-Peter, Dr., 69214 Eppelheim (DE); Tilz, Wolfgang, 68723 Schwetzingen (DE)
(74) Vertreter: Grünecker, Kinkeldey, Stockmair & Schwanhäusser Anwaltssozietät

(57) **Zusammenfassung**

Die Erfindung betrifft ein Verfahren zum Sterilisieren des Anschlussbereichs eines Bag-in-Tank Systems, insbesondere zum Transport für Lebensmittelprodukte, wobei das Bag-in-Tank System einen Tank, einen austauschbar im Inneren des Tanks anordbaren Inliner mit Auslaufstutzen und einer abnehmbaren Ventilvorrichtung zum Anschließen an den Auslaufstutzen aufweist. Um die Sterilisierung zu gewährleisten, ist das Sterilisierungsverfahren mindestens zweistufig, wobei im ersten Schritt die Ventilvorrichtung alleine und im zweiten Schritt die Ventilvorrichtung angebracht am Auslaufstutzen sterilisiert wird. Die Erfindung betrifft ebenso eine entsprechende Anlage zum Sterilisieren des Anschlussbereichs eines Bag-in-Tank Systems.

## Beschreibung

Die Erfindung betrifft ein Verfahren zum Sterilisieren des Anschlussbereichs eines Bag-in-Tank Systems, insbesondere zum Transport für Lebensmittelprodukte, wobei das Bag-in-Tank System einen Tank, einen austauschbar im Inneren des Tanks anordbaren Inliner mit Auslaufstutzen und einer abnehmbaren Ventilvorrichtung zum Anschließen an den Auslaufstutzen, aufweist. Die Erfindung betrifft weiterhin eine entsprechende Anlage zum Sterilisieren des Anschlussbereichs des Bag-in-Tank Systems.

Bag-in-Tank Systeme werden zum Transport von Lebensmittelprodukten, insbesondere von flüssigen beziehungsweise fließenden Lebensmittelprodukten wie Getränke oder Fruchtzubereitungen, benutzt. Gegenüber den herkömmlichen Transporttanks haben diese Systeme den Vorteil, dass nach der Benutzung der Tank nicht aufwendig gereinigt werden muss, sondern nur ein neuer Inliner eingefügt werden muss. Zwar erfüllen die Innenseiten der Inliner üblicherweise die strengen Hygieneanforderungen, die bei den oben genannten Produktfamilien eingehalten werden müssen, um eine möglichst keimfreie Verpackung garantieren zu können, ist es jedoch auch nötig, neben dem sterilen Innenraum des Inliners, auch die Anschlussbereiche des Bag-in-Tank Systems, über die der Inliner befüllt beziehungsweise später wieder entleert wird, entsprechend zu reinigen und zu entkeimen.

Es ist daher Aufgabe der vorliegenden Erfindung, ein Verfahren zum Sterilisieren des Anschlussbereichs eines Bag-in-Tank Systems und eine entsprechende Anlage zum Sterilisieren bereitzustellen, mit der die hohen Hygieneanforderungen, die bei Lebensmittelprodukten auftreten, gewährleistet werden können.

Diese Aufgabe wird mit dem Verfahren nach Anspruch 1 und der Anlage nach Anspruch 11 gelöst.

Anders als bei dem im Stand der Technik gekannten Tanks, wird ein Bag-in-Tank System aus mehreren Teilen zusammengesetzt. So wird der Inliner im Tank angeordnet und dessen Auslaufstutzen durch eine Öffnung unten am Tank mit Hilfe einer Zentrierscheibe fixiert. Daraufhin wird dichtend auf dem Auslaufstutzen eine Ventilvorrichtung angebracht, die üblicherweise auch am Tank, insbesondere an der Öffnung, befestigt wird. Dank der erfindungsgemäßen zweitstufigen Sterilisation wird es ermöglicht, den Anschlussbereich des Bag-in-Tank Systems sicher und wiederholbar zu sterilisieren.

Insbesondere durch das Sterilisieren der abgenommenen Ventilvorrichtung wird sichergestellt, dass auch schwer zugängliche Bereiche der Ventilvorrichtung, beispielsweise dort, wo die Ventilvorrichtung am Auslaufstutzen des Inliners anliegt, hygienisch einwandfrei sind.

Unter Anschlussbereich des Bag-in-Tank Systems versteht man hier den Bereich des Bag-in-Tank Systems, über den nach der Sterilisierung das Produkt eingefüllt wird, beziehungsweise über den das Produkt wieder entnommen wird. Der Anschlussbereich umfasst somit die Ventilvorrichtung und den Auslaufstutzen des Inliners. Es versteht sich von selbst, dass es sich hier um die Sterilisierung der innenliegenden Bereiche handelt und nicht um die außenliegenden Flächen, die nicht mit dem Produkt in Kontakt kommen. Insbesondere muss auch die enthaltenen Luft sterilisiert werden.

Je nach Hygieneanforderungen kann, anstatt dem Sterilisieren auch ein Desinfizieren an zwei verschiedenen Stationen oder eine Mischung aus Desinfizieren an einer Station und Sterilisieren an einer anderen Station ausreichend sein. Die Erfindung umfasst sowohl die Sterilisierung als auch die Desinfizierung bzw. ein Mischung aus beidem. Üblicherweise versteht man unter Sterilisieren die möglichst vollständige Abtötung von Keimen und Sporen, so dass diese in der Nährlösung Lebensmittel nicht wachsen bzw. sich nicht vermehren können.

Vorteilhafter Weise kann im Schritt a) vor der Sterilisierung die Ventilvorrichtung in eine Haltevorrichtung angeordnet werden und eine Andockvorrichtung angeschlossen werden, wobei Ventilvorrichtung, Haltevorrichtung und Andockvorrichtung eine abgedichtete Einheit bilden und die Sterilisierung über die Andockvorrichtung durchgeführt wird. Mit Hilfe der Haltevorrichtung wird sichergestellt, dass der Ventilträger korrekt behandelt wird, so dass Bedienungsfehler möglichst ausgeschlossen werden können. Auch die Ausbildung einer abgedichteten Einheit aus Andockstation, Ventilträger und Haltevorrichtung dient diesem Zweck, da in der dichten Einheit die Sterilisierungsparameter überwacht und somit auch der Sterilisierungsvorgang kontrolliert werden können.

Bevorzugt kann durch Druckluftbeaufschlagung die Dichtheit der Einheit aus Ventilvorrichtung, Haltevorrichtung und Andockvorrichtung überprüft werden. Diese Dichtheitsprüfung dient dazu, dass die Sterilisierung möglichst fehlerfrei durchgeführt werden kann, um die geforderte Hygiene zu garantieren.

Vorteilhafter Weise kann in Schritt c) die Sterilisierung in der zweiten Sterilisationsstation auch über die Andockvorrichtung durchgeführt werden. In dem beispielsweise die Andockvorrichtung schwenkbar ausgeführt wird, lassen sich die zwei Sterilisationsschritte mit nur einer Andockvorrichtung durchführen, wodurch sich der Aufbau der Sterilisierungsanlage vereinfacht.

Gemäß einer bevorzugten Ausführungsform kann in Schritt b) die Ventilvorrichtung zusammen mit der Andockvorrichtung am Auslaufstutzen angebracht werden. Dies hat den Vorteil, dass die Ventilvorrichtung nicht noch einmal von der Andockvorrichtung gelöst werden muss und somit vom Anwender nicht mehr in die Hand genommen werden muss. Somit wird eine Wahrscheinlichkeit einer Verunreinigung nach dem ersten Sterilisationsschritt verringert.

Bevorzugt kann die Sterilisierung der Ventilvorrichtung durch Dampfaufschlagung, insbesondere mit Dampf von mindestens 110°C durchgeführt werden. Dank der Sterilisierung in der gasförmigen Phase werden auch schwer zugängliche Bereiche der Ventilvorrichtung sicher sterilisiert.

Bevorzugt kann die Sterilisierung in Schritt a) durch Temperaturmessung des auftretenden Kondensats kontrolliert werden. Da der heiße Dampf an den kalten Oberflächen der Ventilvorrichtung kondensiert, gibt die Temperatur des Kondensats Auskunft darüber, wie warm die Wände sind. Ab einer gewissen Kondensattemperatur kann dann darauf geschlossen werden, dass der Sterilisierungsvorgang wirksam vorgenommen wird. Durch die laufende Temperaturprüfung kann auch kontrolliert werden, dass die Sterilisierung ausreichend lange bei der gewollten Temperatur durchgeführt wird.

Gemäß einer bevorzugten Ausführungsform kann die Sterilisierung im Schritt c) mit Wasserstoffperoxyd durchgeführt werden. Wasserstoffperoxyd hat den Vorteil, dass die entkeimende Wirkung schon bei geringen Temperaturen erzielt werden kann. Somit kann auch sterilisiert werden, wenn hitzeempfindliche Materialien, wie beispielsweise Kunststoffe, vorliegen. Dabei muss jedoch berücksichtigt werden, dass sich bei niedrigen Temperaturen die Sterilisierungsdauer verlängert. Das Wasserstoffperoxyd kann gasförmig oder flüssig eingesetzt werden, wobei das flüssige Wasserstoffperoxyd beim Kontakt mit den im Schritt a) aufgeheizten Wänden verdampfen kann, um somit die Luft zu entkeimen.

Gemäß einer weiteren Ausführungsform kann das erfindungsgemäße Verfahren die weiteren Schritte d) Transport des Bag-in-Tank Systems zu einer Befüllstation, e) Sterilisieren produktführender Bereiche der Befüllstation und/oder des Anschlussbereichs des Bag-in-Tank Systems in einer dritten Sterilisationsstation und f) Befüllen des Inliners durch die Befüllstation aufweisen. Da beim Anschließen der Befüllstation an die geschlossene Ventilvorrichtung der Weg den das Produkt bis zur Ventilvorrichtung zurücklegen eventuell verunreinigte Luft aufweisen kann bzw. der produktführende Kanal und die Außenseite der Ventilvorrichtung kontaminiert sein können, wird erfindungsgemäß dieser Bereich in der dritten Station sterilisiert. Werden die vorbereiteten Bag-in-Tank Systeme nicht sofort befüllt, sondern erst gelagert oder an einem anderen Ort befüllt, so ist es außerdem vorteilhaft den Anschlussbereich, also die Ventilvorrichtung, bei offenem Ventil, und den Auslaufstutzen im zusammengesetzten Zustand, kurz vor der Befüllung zu sterilisieren, um die Keimfreiheit zu garantieren. Somit wird gewährleistet, dass das Produkt keimfrei in den Inliner gelangen kann.

Vorzugsweise kann dabei die Sterilisierung des Anschlussbereichs des Bag-in-Tank Systems durch Dampfbeaufschlagung, insbesondere mit Dampf von mindestens 110°C durchgeführt werden.

Die Erfindung betrifft ebenso eine Anlage zum Sterilisieren des Anschlussbereichs eines Bag-in-Tank Systems, insbesondere zum Transport für Lebensmittelprodukte, wobei das Bag-in-Tank System einen Tank, einen austauschbar im Inneren des Tank anordbaren Inliner mit Auslaufstutzen und eine abnehmbaren Ventilvorrichtung zum Anschließen an den Auslaufstutzen aufweist, mit:
a) einer ersten Sterilisationsstation zum Sterilisieren der vom Tank abgenommenen Ventilvorrichtung, und
b) einer zweiten Sterilisationsstation zum Sterilisieren der Ventilvorrichtung und des Auslaufstutzens im zusammengesetztem Zustand.

Mit dieser Anlage kann das erfindungsgemäßen Verfahrens realisiert werden und somit auch die Vorteile des zweistufigen Verfahrens.

Bevorzugt kann die erste Sterilisationsstation eine Haltevorrichtung zum Aufnehmen der Ventilvorrichtung und eine Andockvorrichtung, die so ausgebildet ist, dass über sie die Sterilisierung durchgeführt wird, aufweisen. Dieser Aufbau ermöglicht eine betriebssichere Durchführung der Sterilisierung.

Gemäß einer bevorzugten Ausführungsform kann die Andockvorrichtung so ausgebildet sein, dass bei geöffnetem Ventil, die Haltevorrichtung, die Ventilvorrichtung und die Andockvorrichtung dichtend miteinander verbunden sind.

Vorteilhafter Weise kann die Haltevorrichtung ein Mittel zur Temperaturmessung aufweisen. Dank der Temperaturmessung kann gewährleistet werden, dass der Sterilisierungsvorgang bei den gewünschten Bedingungen - Temperatur und Dauer - durchgeführt wird.

Gemäß einer bevorzugten Ausführungsform kann die Andockstation eine Dampfzuführeinheit zum Sterilisieren und eine Druckluftzuführeinheit zur Überprüfung der dichten Anordnung der Haltevorrichtung, der Ventilvorrichtung und der Andockvorrichtung aufweisen. Durch die Druckluftbeaufschlagung kann die Dichtheit einfach kontrolliert werden und anschließend betriebssicher mit dem Dampf sterilisiert werden.

Bevorzugt kann die zweite Sterilisationsstation der Andockstation der ersten Sterilisationsstation umfassen. Dies vereinfacht den Aufbau der ersten und zweiten Sterilisationsstation, da es beispielsweise ausreicht die Andockstation schwenkbar auszubilden.

Vorteilhafterweise kann die Andockstation so ausgebildet sein, dass die Andockvorrichtung mit der Ventilvorrichtung am Auslaufstutzen anbringbar ist. Dies ermöglicht, dass die Ventilvorrichtung nach der Sterilisation in der ersten Sterilisationsstation nicht noch einmal vom Benutzer in die Hand genommen werden muss, sondern zusammen mit der Andockstation von der ersten Sterilisationsstation zur zweiten Sterilisationsstation gebracht werden kann.

Gemäß einer bevorzugten Ausführungsform kann die Andockstation eine Wasserstoffperoxydzuführeinheit aufweisen. Durch den kombinierten Einsatz von Dampf und Wasserstoffperoxid wird eine ausreichende Entkeimung gewährleistet.

In einer bevorzugten Ausführungsform kann die erfindungsgemäße Anlage eine Befüllstation zum Befüllen des Inliners, die eine dritte Sterilisationsstation zum Sterilisieren produktführender Bereiche der Befüllstation und/oder des Anschlussbereichs umfasst, aufweisen. Da beim Anschließen der Befüllstation an die geschlossene Ventilvorrichtung der Weg den das Produkt bis zur Ventilvorrichtung zurücklegen eventuell verunreinigte Luft aufweisen kann bzw. der produktführende Kanal und die Außenseite der Ventilvorrichtung kontaminiert sein kann, wird erfindungsgemäß dieser Bereich in der dritten Station sterilisiert. Werden die vorbereiteten Bag-in-Tank Systeme nicht sofort befüllt, sondern erst gelagert oder an einem andren Ort befüllt, so ist es vorteilhaft, den Anschlussbereich, also die Ventilvorrichtung und den Auslaufstutzen im zusammengesetzten Zustand, kurz vor der Befüllung zu sterilisieren, um die Keimfreiheit zu garantieren.

Nachfolgend werden anhand der Figuren bevorzugte Ausführungsformen der Erfindung und deren Vorteile näher erläutert. Es zeigen:
- Figur 1:: eine Querschnittsansicht eines Bag-in-Tank Transportsystems,
- Figur 2:: schematisch die erste Sterilisationsstation einer erfindungsgemäßen Ausführung der Anlage zum Sterilisieren des Anschlussbereichs eines Bag-in-Tank Systems,
- Figur 3:: schematisch die zweite Sterilisationsstation,
- Figur 4:: eine zweite Ausführungsform der Anlage zum Sterilisieren mit drei Sterilisationsstationen,
- Figur 5:: ein Ablaufdiagramm einer ersten Ausführungsform des erfindungsgemäßen Verfahren zum Sterilisieren und
- Figur 6:: ein Ablaufdiagramm einer zweiten Ausführungsform des erfindungsgemäßen Verfahren zum Sterilisieren.

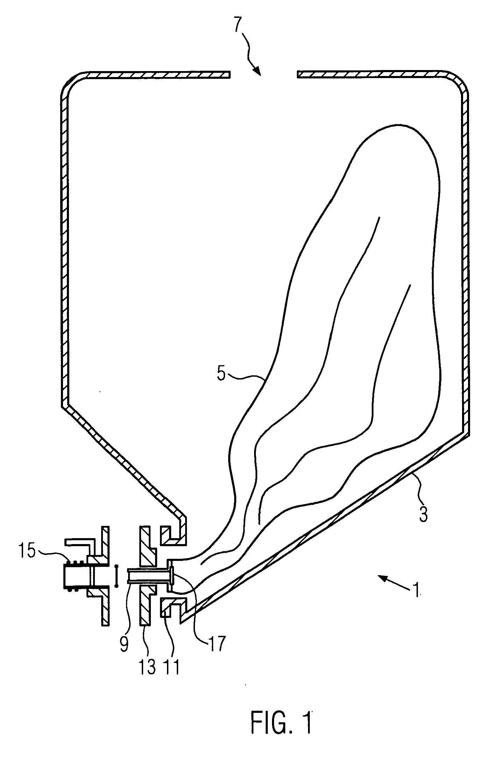

Figur 1 zeigt schematisch ein Bag-in-Tank System 1 zum Transport von Lebensmittelprodukten, wie beispielsweise Saft- oder Fruchtzubereitungen. Im Vergleich zu herkömmlichen Tanks haben die Bag-in-Tanks Systeme 1 den Vorteil, dass nach der Benutzung der Tank nicht mehr aufwendig gereinigt und sterilisiert werden muss, da sich im Inneren des Tanks 3 ein Inliner 5 befindet, der nach der Benutzung durch einen neuen ersetzt wird. Nach der Benutzung wird der Inliner 5 durch eine Öffnung 7 im Tank 3 aus dem Inneren des Tanks entfernt und durch einen neuen Inliner 5 ersetzt. Der neue Inliner 5 wird so in den Tank 3 eingelegt, dass ein Auslaufstutzen 9 an einer entsprechenden Öffnung 11 im Tank 3 herausschaut. Mit Hilfe einer Zentrierscheibe 13 wird der Auslaufstutzen 9 dann in der Öffnung 11 zentriert und schließlich wird eine Ventilvorrichtung 15 dichtend auf den Auslaufstutzen 9 aufgesetzt. Um die Keimfreiheit des Inneren des Inliners 5 zu gewährleisten, ist üblicherweise noch eine Berstmembran beziehungsweise Berstscheibe 17 am Auslaufstutzen 9 angeordnet. Diese Berstmembran 17 bedeckt den Ein- beziehungsweise Auslaufkanal des Auslaufstutzens 9. Beim Einfüllen des Produktes reißt diese Membran fetzenfrei auf, so dass nur das Produkt ins Innere des Inliners 5 gelangt.

Nachfolgend versteht man unter dem Anschlussbereich eines Bag-in-Tank Systems 1 die Ventilvorrichtung 15 und den Auslaufstutzen 9 des Inliners 5 bis zur Berstmembran 17. Die Ventilvorrichtung 15 wird nicht nur am Auslaufstutzen 9 angebracht, sondern auch, beispielsweise mit Hilfe von Klemmschellen, am Tank 3 befestigt.

Die erläuterte Ausführungsform eines Bag-in-Tank Systems stellt hier nur ein Beispiel dar. Das nachfolgend beschriebene Verfahren und die Anlage zum Sterilisieren des Anschlussbereichs des Bag-in-Tank Systems sind auch auf andere Bag-in-Tank Systeme anwendbar. Beispielsweise könnte die Öffnung 11 des Tanks 3 so groß gewählt werden, dass der Inliner 5 durch diese eingeführt werden kann, so dass auf eine Öffnung 7 im Deckelbereich des Tanks 3 verzichtet werden kann.

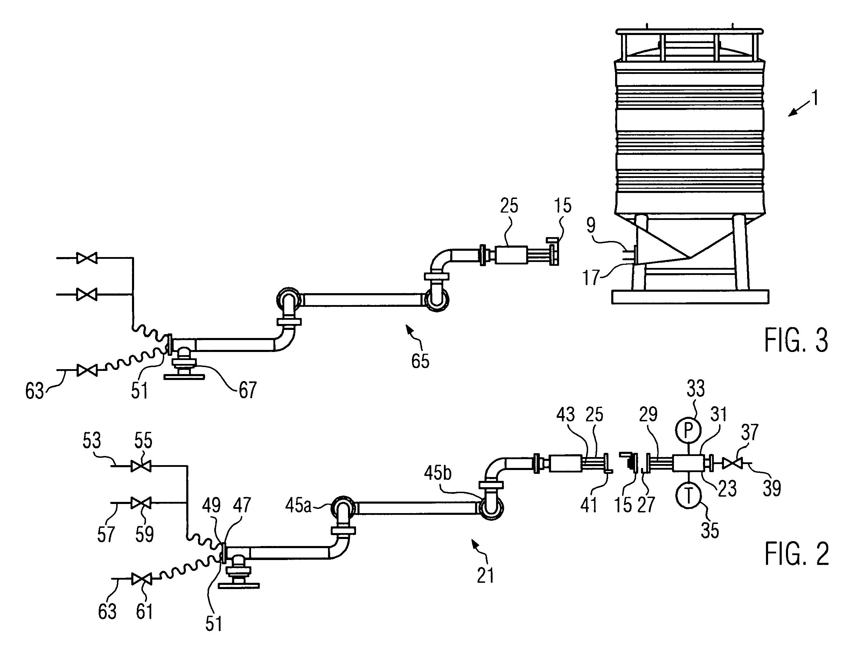

Figur 2 zeigt eine Ausführungsform einer ersten Sterilisationsstation 21 einer erfindungsgemäßen Anlage zum Sterilisieren des Anschlussbereichs eines Bag-in-Tank Systems, wie beispielsweise in Figur 1 dargestellt. Hierzu wird nachfolgend Bezug auf die Elemente des Bag-in-Tank Systems 1 der Figur 1 genommen. Die einzelnen Elemente und deren Funktionsweise, wie beispielsweise der Ventilvorrichtung 15 oder des Anschlussbereichs 9, werden daher nicht mehr im Einzelnen erläutert.

Die erste Sterilisationsstation 21 dient zum Sterilisieren der vom Tank 3 abgenommenen Ventilvorrichtung 15. Die erste Sterilisationsstation 21 umfasst eine Haltevorrichtung 23 zum Aufnehmen der Ventilvorrichtung 15 und eine Andockvorrichtung 25, über die die Sterilisierung durchgeführt wird. Die Haltevorrichtung 23 umfasst eine Halterung 27, in die die Ventilvorrichtung 15 gestellt werden kann. Weiterhin umfasst die Haltevorrichtung einen Kanal 29, der fluchtend mit der gehaltenen Ventilvorrichtung 15 ausgebildet ist. Dieser Kanal endet in einer Analysekammer 31 an die ein Druckmessgerät 33 und ein Temperatursensor 35 angeschlossen ist. An die Kammer 31 schließt sich ein Ventil 37 an, das einen Ablauf 39 sperren oder öffnen kann.

Die Andockvorrichtung 25 hat einen Anschlussbereich 41, über den die Andockvorrichtung 25 zusammen mit der Ventilvorrichtung 15 und der Haltevorrichtung 29 eine dichte Einheit bilden kann. Nicht gezeigt sind Greifer am Anschlussbereich 41, die die drei Elemente zusammenschieben um die abdichtende Einheit zu bilden. Wie die Haltevorrichtung 29 weist auch die Andockstation 25 einen Kanal 43 auf, der fluchtend mit der Ventilvorrichtung 15 und dem Kanal 29 ist, wenn die drei Elemente zusammengeführt sind. Die Haltevorrichtung 25 weist Gelenke 45a und 45b auf, so dass die Andockvorrichtung 25 auf die in der Haltevorrichtung 29 angeordnete Ventilvorrichtung 15 hinbewegt werden kann. An ihrem von der Ventilvorrichtung 15 abgewandten Ende 47 weist die Andockvorrichtung 25 zwei Anschlüsse 49 und 51 auf. Am Anschluss 49 ist eine Dampfzuführeinheit 53, insbesondere für Wasserdampf, über ein Ventil 55 und eine Druckluftzuführeinheit 57 über ein weiteres Ventil 59 an der Andockvorrichtung 25 angeschlossen.

Am zweiten Anschluss 51 ist über ein weiteres Ventil 61 eine Wasserstoffperoxydzuführeinheit 63 angeschlossen.

Wie später im Zusammenhang mit dem erfindungsgemäßen Verfahren näher erläutert wird, wird in der ersten Sterilisationsstation die Druckluftzuführeinheit 57 und die Dampfzuführeinheit- 55 benutzt. Dabei dient die Druckluft zur Überprüfung der dichten Anordnung der Andockvorrichtung 25, der Ventilvorrichtung 15 und der Haltevorrichtung 25 und der Dampf zum Sterilisieren. Die Wasserstoffperoxydzuführeinheit 63 wird in Zusammenhang mit der zweiten Sterilisationsstation, die in der Figur 3 erläutert wird, benötigt. Da jedoch vorteilhafter Weise die Andockvorrichtung 25 sowohl in der ersten Sterilisationsstation als auch in der noch zu beschreibenden zweiten Sterilisationsstation benutzt wird, liegen zwei Anschlüsse 49 und 51 vor.

Im vorliegenden Ausführungsbeispiel wird Dampf, insbesondere Wasserdampf, und/oder Wasserstoffperoxyd zum Sterilisieren benutzt. Dem Fachmann sind jedoch auch alternative Produkte, wie beispielsweise Alkohol, Essigsäure insbesondere auch in Dampfform, sowie generell alle für Lebensmittel zugelassenen Desinfektionsmittel, die auf über 110°C erhitzt werden können, bekannt. Diese können ebenso erfindungsgemäß verwendet werden.

Figur 3 zeigt die zweite Sterilisationsstation 67 der erfindungsgemäßen Anlage zum Sterilisieren des Anschlussbereichs eines Bag-in-Tank Systems. Die zweite Sterilisationsstation 67 dient zum Sterilisieren der Ventilvorrichtung 15 und des Auslaufstutzens 9 des Inliners 5 im zusammengesetztem Zustand. Dies ist nötig, da beim Andocken verunreinigte Luft in den Auslaufstutzen 9 gelangen kann. In Figur 3 ist der Zustand gezeigt, in dem die Ventilvorrichtung 15 und der Auslaufstutzen 9 noch nicht aneinander angebracht sind.

In dieser Ausführungsform der erfindungsgemäßen Anlage zum Sterilisieren des Anschlussbereichs eines Bag-in-Tank Systems benutzt die zweite Sterilisationsstation 65 die Andockstation 25 der ersten Sterilisationsstation 21, wodurch der Aufbau der Anlage vereinfacht wird. Die in Figur 3 dargestellte Andockstation 25 umfasst somit die gleichen Elemente, die schon im Zusammenhang mit der Figur 2 beschrieben und erläutert wurden.

Zur Durchführung der Sterilisation wird die Andockvorrichtung 25 zusammen mit der Ventilvorrichtung 15 auf den Auslaufstutzen 9 zu bewegt und mit diesem dichtend verbunden. Die Sterilisierung findet dann über den zweiten Anschluss 51 statt. Zur Sterilisierung wird erfindungsgemäß Wasserstoffperoxyd (H₂O₂) über die Wasserstoffperoxydzuführeinheit 63 zugeführt. Wird ein Bag-in-Tank System 1, wie in Figur 1 dargestellt, benutzt, muss darauf geachtet werden, dass es bei der Sterilisierung an der zweiten Sterilisationsstation 65 durch das Einlaufen des Sterilisationsmittels nicht zu einem Aufreißen beziehungsweise Aufplatzen der Berstmembran 17 am Auslaufstutzen kommt. Da es sich bei Berstmembranen meist um Kunststofffolien, z.B. aus PE, HDPE, PP, HDOO oder PET, handelt, muss außerdem beachtet werden, dass in einem Temperaturbereich gearbeitet wird, in dem der Kunststoff nicht beschädigt, beispielsweise aufgeweicht, wird.

Bevorzugt wird flüssiges Wasserstoffperoxyd in den Auslaufstutzen eingesprüht, an den noch vom ersten Sterilisationsvorgang heißen Wänden verdampft dann das H₂O₂. Es kann jedoch auch gleich mit gasförmigem Wasserstoffperoxyd gearbeitet werden.

Damit die Andockvorrichtung 25 sowohl in der ersten Sterilisationsstation 21 als auch in der zweiten Sterilisationsstation 65 (Figur 2 und Figur 3) benutzt werden kann, muss die Andockvorrichtung 25 mobil ausgestaltet sein. In dieser Ausführungsform kann die Andockvorrichtung 25 über ein Gelenk 67 in der Ebene senkrecht zur Zeichenebene bewegt werden.

Wie in Figur 3 dargestellt, verbleibt nach der Sterilisierung der Ventilvorrichtung 15 die Ventilvorrichtung 15 an der Andockvorrichtung 25. Dies verhindert, dass die Ventilvorrichtung 15 noch einmal vom Benutzer in die Hand genommen werden muss.

Gemäß einer weiteren Ausführungsform könnte jede Sterilisationsstation 21, 65 anstatt nur einer gemeinsamen Andockvorrichtung 25 ihre eigene Andockvorrichtung aufweisen. In diesem Fall bräuchte die Andockvorrichtung der ersten Sterilisationsstation 21 nur einen Anschluss über den Dampf beziehungsweise Druck eingeleitet werden kann und die Andockvorrichtung der zweiten Sterilisationsstation 65 ebenso nur einen Anschluss über den Wasserstoffperoxyd eingeleitet werden kann.

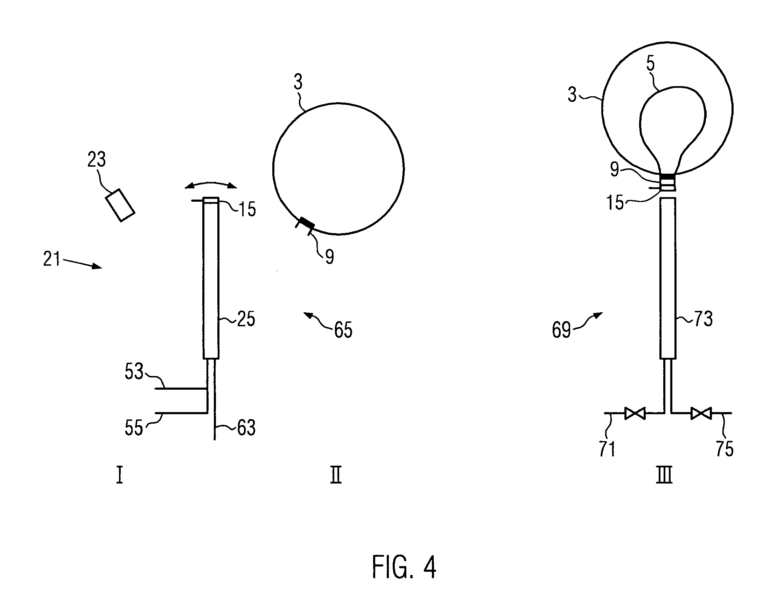

Figur 4 zeigt schematisch eine zweite Ausführungsform der Anlage zum Sterilisieren des Anschlussbereichs eines Bag-in-Tank Systems. Diese Anlage weist drei Sterilisationsstationen 21, 65 und 69 auf. Die erste und zweite Basisstation 21, 65 entsprechen den in den Figuren 2 und 3 dargestellten Sterilisationsstationen. Schematisch dargestellt ist die Andockvorrichtung 25 mit der Dampfzuführeinheit 53, der Druckluftzuführeinheit 55 und der Wasserstoffperoxydzuführeinheit 63 an ihrem einen Ende und einer Ventilvorrichtung 15 an ihrem anderen Ende. Der Pfeil deutet an, dass die Andockvorrichtung 25 sowohl in der ersten Sterilisationsstation 21 als auch der zweiten Sterilisationsstation 63 verwendet werden kann. In der ersten Sterilisationsstation 21 ist die Haltevorrichtung 23 angedeutet und in der zweiten Sterilisationsstation 63 ein Bag-in-Tank System 1 mit Tank 3 und Auslaufstutzen 9, wie schon im Zusammenhang mit der Figur 1 erläutert.

Zusätzlich zu den ersten beiden Sterilisationsstationen 21, 63 der ersten Ausführungsform weist die zweite Ausführungsform nun auch noch eine dritte Sterilisationsstation 69 auf, die sich eventuell an einem anderen Ort befinden kann. Diese dritte Sterilisationsstation 69 befindet sich an einer Befüllstation zum Befüllen des Inliners 5, der sich im Inneren des Tanks 3 des Bag-in-Tank Systems 1 befindet und dient dazu den Bereich von einer Produktzuführeinheit 71 bis zum geschlossenen Ventil der Ventilvorrichtung 15 zu sterilisieren. Je nachdem wie lange das Bag-in-Tank System gelagert wurde, kann es eventuell aber auch nötig sein noch einmal den Anschlussbereich, der sich aus der Ventilvorrichtung 15 und dem Anschlussstutzen 9 zusammensetzt, vor dem Befüllen zu reinigen, insbesondere zu sterilisieren. Zur Sterilisierung wird über eine weitere Andockvorrichtung 73 dem Anschlussbereich Dampf, insbesondere Wasserdampf, über eine Dampfzuführeinheit 75 zugeführt. Alternativ kann, wie in der ersten und/oder zweiten Sterilisationsstation, auch Wasserstoffperoxyd benutzt werden. Nach dem dritten Sterilisationsvorgang kann dann das Produkt über die Produktzuführeinheit 71 und die sterilisierte Andockvorrichtung 73 dem Inliner zugeführt werden. Hierzu muss, falls vorhanden, die Berstmembran 17 durchbrochen werden.

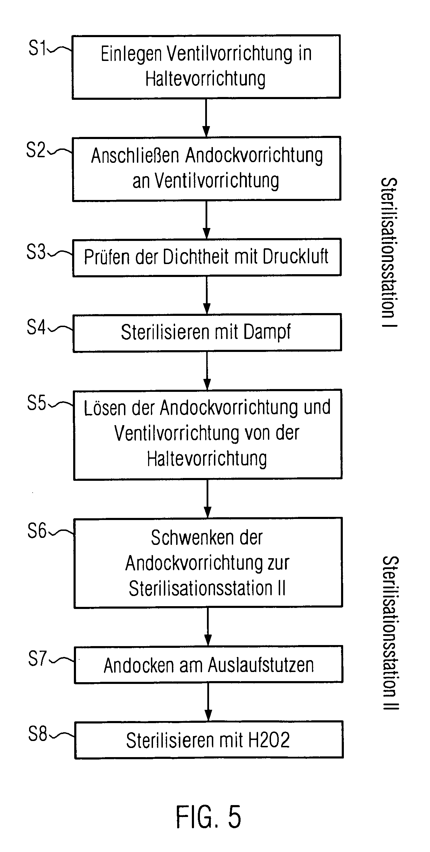

Figur 5 zeigt ein Ablaufdiagramm einer ersten Ausführungsform des erfinderischen Verfahrens zum Sterilisieren des Anschlussbereichs eines Bag- in-Tank Systems 1, wie in Figur 1 dargestellt. Das Ablaufdiagramm erläutert weiterhin die Funktionsweise der oben beschriebenen erfindungsgemäßen Anlage, wie sie in den Figuren 2 und 3 dargestellt ist.

Die Schritte S1-S5 finden an der Sterilisationsstation I, wie sie in der Figur 2 gezeigt ist, statt. Im Schritt S1 wird die Ventilvorrichtung 15 in den Halter 27 der Haltervorrichtung 23 eingelegt. Im anschließenden Schritt 2 wird dann die Andockvorrichtung 25 auf die Ventilvorrichtung 15 zu bewegt und so angeschlossen, dass der Kanal 43 der Andockvorrichtung 25, die Öffnung der Ventilvorrichtung 15 und der Kanal 29 der Haltevorrichtung 23 fluchtend angeordnet sind. Der Anschluss soll so stattfinden, dass die Andockvorrichtung 25, die Ventilvorrichtung 15 und die Haltevorrichtung 23 eine abgedichtete Einheit bilden. Um dies zu gewährleisten werden die in der Figur 2 nicht dargestellten Greifer der Andockvorrichtung 5 benutzt, die nach dem Anschließen die Andockvorrichtung, die Ventilvorrichtung und die Haltevorrichtung zusammenschieben. Die Dichtheit wird mit Dichtungsringen gewährleistet.

Die Dichtheit wird dann im Schritt S3 mit Hilfe von Druckluft überprüft. Hierzu wird das Ventil 59 geöffnet und dadurch die Einheit aus Andockstation 25, Ventilvorrichtung 15 und Haltevorrichtung 23 mit Druckluft beaufschlagt. Durch Messung des Drucks mit dem Druckmessgerät 33 wird dann ermittelt, ob die drei Einheiten dicht miteinander verbunden sind. Da mit der Anlage eine Sterilisation durchgeführt werden soll, wird in der Druckluftzuführeinheit 57 die unter Druck stehende Luft vorgereinigt.

Nachdem gewährleistet wurde, dass die Andockstation 25, die Ventilvorrichtung 15 und die Haltevorrichtung 23 dicht miteinander verbunden sind, wird im Schritt S4 die eigentliche Sterilisierung mit Dampf, insbesondere Wasserdampf, durchgeführt. Dazu wird Ventil 59 geschlossen und Ventil 55 geöffnet, so dass nun das Innere der Andockvorrichtung 25, der Ventilvorrichtung 15 und der Haltevorrichtung 23 mit heißem Dampf beaufschlagt werden kann. Insbesondere hat es sich gezeigt, dass Dampf mit mindestens 110°C, bevorzugt mit 120°C, die in der Sterilisationsstation 21 gewünschte Sterilisierung der Ventilvorrichtung 15 durchführen kann.

Zur Kontrolle des Sterilisierungsvorgangs wird mit Hilfe des Temperatursensors 35 die Temperatur des in der Haltevorrichtung 23 auftretenden Kondensats gemessen. Sobald diese Temperatur einen gewissen Schwellenwert überschreitet, läuft die Sterilisationszeit los. Wird während der Sterilisierung dieser Schwellwert unterschritten, so bleibt die Zeit stehen. Wird dann wiederum der Schwellwert erreicht, beginnt die Sterilisationszeit von vorne. Dank des Ablaufs 39 kann das Kondensat bei offenem Ventil 37 ablaufen. Nach Ablauf der nötigen Sterilisationsdauer wird dann das Ventil 55 geschlossen und die Andockvorrichtung 25, die Ventilvorrichtung 15 und die Haltevorrichtung 23 mit Luft, insbesondere trockener Luft, beaufschlagt.

Bevor nun die Ventilvorrichtung 15 am Tank 3 des Bag-in-Tank Systems 1 angebracht werden kann, wird im Schritt S5 die Andockvorrichtung 25 zusammen mit der Ventilvorrichtung 15 von der Haltevorrichtung 23 gelöst. Um dies zu ermöglichen, weist die Andockvorrichtung 25 ebenso eine Halterung (nicht dargestellt) für die Ventilvorrichtung 15 auf. Nach der Trennung von der Haltevorrichtung kann eine Schutzkappe auf die Ventilvorrichtung angebracht werden.

In Schritt S6 wird nun die Andockvorrichtung 25 zusammen mit der Ventilvorrichtung 15 zur Sterilisationsstation 65 hingeschwenkt. Im Schritt S7 wird die Ventilvorrichtung 15 am Auslaufstutzen 9 angedockt (siehe Figur 3), eventuell nach Abnahme der Schutzkappe. Auch diese Verbindung ist naturgemäß dicht. Hierbei kann die Ventilvorrichtung 15 entweder am Auslaufstutzen 9 oder direkt am Tank 3 des Bag-in-Tank Systems 1 befestigt werden. Hierzu geeignet sind beispielsweise Klemmschellen.

Im Schritt S8 wird dann der Anschlussbereich, also die Ventilvorrichtung 15 zusammen mit dem Anschlussstutzen 9 bis zur Berstmembran 17 sterilisiert. Dazu wird über den zweiten Anschluss 51 bei offenem Ventil 61 aus der Wasserstoffperoxydzuführeinheit 63 flüssiges oder gasförmiges Wasserstoffperoxyd eingeleitet. Flüssiges Wasserstoffperoxyd kann dabei an den noch heißen Wänden verdampfen. Dieser Sterilisierungsvorgang endet nach einer vorbestimmten Zeit. Ist dieser zweite Schritt zu Ende, wird die bis dahin offene Ventilvorrichtung 15 geschlossen und die Andockvorrichtung 25 von der Ventilvorrichtung 15 getrennt.

Um den nun offenen Bereich sauber zu halten, kann die Ventilvorrichtung 15 mit Hilfe eines Deckels oder einer Kappe geschützt werden.

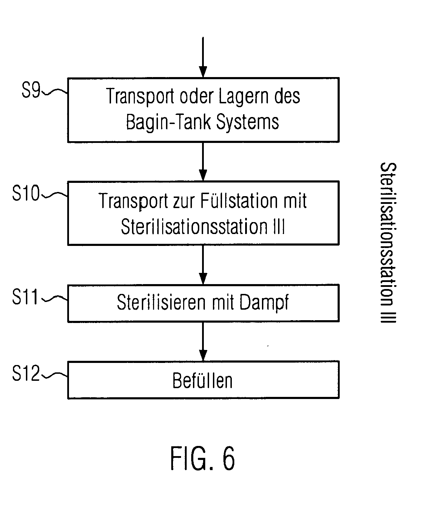

Figur 6 zeigt eine zweite Ausführungsform des erfindungsgemäßen Verfahrens. Die in Figur 6 dargestellten Schritte S9-S12 schließen sich dabei an die in Figur 5 dargestellten Schritte S1-S8 an. Erläutert wird gleichzeitig die Funktionsweise der dritten Sterilisationsstation 69 der zweiten Ausführungsform der erfindungsgemäßen Anlage. Nach der zweiten Sterilisierung in der Sterilisationsstation 65 wird üblicherweise das Bag-in-Tank System 1 an einen anderen Ort gebracht oder gelagert (Schritt S9). Im Schritt S10 wird das Bag-in-Tank System 1 dann zu einer Befüllstation transportiert. Erfindungsgemäß weist diese Befüllstation, wie oben beschrieben, eine dritte Sterilisationsstation 69 auf.

Im Schritt S11 wird dann die Andockstation 73 bis zur geschlossenen Ventilvorrichtung 15 sterilisiert. In dieser Ausführungsform wird hierzu bei offenem Ventil 55 der Dampfzuführeinheit 75 die Andockstation 73 und die geschlossene Ventilvorrichtung 15 mit Dampf, insbesondere mit einer Temperatur von mehr als 110°C, bevorzugt von 120°C, beaufschlagt. Eventuell kann dann der Anschlussbereich, also Ventilvorrichtung 15 und Anschlussstutzen 9 bis zur Berstmembran 17 zur erneuten Entkeimung bei offenem Ventil der Ventilvorrichtung 15 noch einmal sterilisiert werden. Alternativ zum Dampf, insbesondere Wasserdampf, könnte wiederum Wasserstoffperoxyd oder eines der oben erwähnten Mittel benutzt werden.

Nach der Sterilisierung im Schritt S11 kommt es dann schließlich im Schritt S12 zur Befüllung des Inliners 5, der sich im Tank 3 des Bag-in-Tank Systems 1 befindet. Hierzu wird das Ventil der Produktzuführeinheit 71 geöffnet. Durch den durch den Produktstrom auf die Berstmembran 17 entstehenden Druck platzt diese auf und das Produkt läuft in den Inliner 5 ein.

Mit dem dargestellten Verfahren und den Anlagen zur Sterilisierung des Anschlussbereichs eines Bag-in-Tank Systems wird ein möglichst keimfreies Befüllen gewährleistet. Alternativ, kann die gleiche Anlage jedoch auch zum Desinfizieren benutzt werden, wenn die Hygieneanforderungen an das Produkt geringer sind.

Die Trennung in mehrere Sterilisationsstationen 21, 65, 69, wobei in der ersten die Ventilvorrichtung alleine, in der zweiten die Ventilvorrichtung angebracht am Auslaufstutzen und in der dritten noch einmal kurz vor der Befüllung die Ventilvorrichtung angebracht am Auslaufstutzen sterilisiert wird, wird sichergestellt, dass auch schwer zugängliche Bereiche, insbesondere in der Ventilvorrichtung 15 ausreichend entkeimt werden. Gleichzeitig wird ermöglicht, dass alle Bereich, die mit dem Produkt in Kontakt treten, ausreichend entkeimt werden.

## Patentansprüche

1. Verfahren zum Sterilisieren des Anschlussbereichs eines Bag-in-Tank Systems (1), insbesondere zum Transport für Lebensmittelprodukte, wobei das Bag-in-Tank System einen Tank (3), einen austauschbar im Inneren des Tanks anordbaren Inliner (5) mit Auslaufstutzen (9) und eine abnehmbare Ventilvorrichtung (15) zum Anschließen an den Auslaufstutzen (9) aufweist, mit den Schritten:
d) Sterilisieren der vom Tank (3) abgenommenen Ventilvorrichtung (15) in einer ersten Sterilisationsstation (21),
e) Anbringen der Ventilvorrichtung (15) am Auslaufstutzen (9), und
f) Sterilisieren der Ventilvorrichtung (15) und des Auslaufstutzens (9) im zusammengesetzten Zustand, der den Anschlussbereich bildet, in einer zweiten Sterilisationsstation (65).

2. Verfahren nach Anspruch 1, wobei im Schritt a) vor der Sterilisierung die Ventilvorrichtung (15) in einer Haltevorrichtung (23) angeordnet wird und an eine Andockvorrichtung (25) angeschlossen wird, wobei Ventilvorrichtung (15), Haltevorrichtung (23) und Andockvorrichtung (25) eine dichte Einheit bilden und die Sterilisierung über die Andockvorrichtung (25) durchgeführt wird.

3. Verfahren nach Anspruch 2, wobei durch Druckluftbeauschlagung die Dichtheit der Einheit aus Ventilvorrichtung (15), Haltevorrichtung (23) und Andockvorrichtung (25) überprüft wird.

4. Verfahren nach einem der Ansprüche 1 bis 3, wobei im Schritt c) die Sterilisierung über die Andockvorrichtung (25) durchgeführt wird.

5. Verfahren nach einem der Ansprüche 2 bis 4, wobei im Schritt b) die Ventilvorrichtung (15) zusammen mit der Andockvorrichtung (25) am Auslaufstutzen (9) angebracht wird.

6. Verfahren nach einem der Ansprüche 1 bis 5, wobei die Sterilisierung der Ventilvorrichtung (15) durch Dampfbeaufschlagung, insbesondere mit Dampf von mindestens 110°C, durchgeführt wird.

7. Verfahren nach Anspruch 6, wobei die Sterilisierung im Schritt a) durch Temperaturmessung des auftretenden Kondensats kontrolliert wird.

8. Verfahren nach einem der Ansprüche 1 bis 7, wobei die Sterilisierung im Schritt c) mit Wasserstoffperoxid durchgeführt wird.

9. Verfahren nach einem der Ansprüche 1 bis 8, mit den zusätzlichen Schritten
d) Transport des Bag-in-Tank Systems (1) zu einer Befüllstation,
e) Sterilisieren produktführender Bereiche (73) der Befüllstation des Anschlussbereichs des Bag-in-Tank Systems (1) in einer dritten Sterilisationsstation (69) und
f) Befüllen des Inliners (5) durch die Befüllstation.

10. Verfahren nach Anspruch 9, wobei die Sterilisierung des Anschlussbereichs des Bag-in-Tank Systems (1) durch Dampfbeaufschlagung, insbesondere mit Dampf von mindestens 110°C, durchgeführt wird.

11. Anlage zum Sterilisieren des Anschlussbereichs eines Bag-in-Tank Systems, insbesondere zum Transport für Lebensmittelprodukte, wobei das Bag-in-Tank System (1) einen Tank (3), einen austauschbar im Inneren des Tanks anordbaren Inliner (5) mit Auslaufstutzen (9) und eine abnehmbare Ventilvorrichtung (15) zum Anschließen an den Auslaufstutzen (9) aufweist, mit:
d) einer ersten Sterilisationsstation (21) zum Sterilisieren der vom Tank abgenommenen Ventilvorrichtung (15), und
e) einer zweiten Sterilisationsstation (65) zum Sterilisieren der Ventilvorrichtung (15) und des Auslaufstutzens (9) im zusammengesetzten Zustand.

12. Anlage nach Anspruch 11, wobei die erste Sterilisationsstation (21) eine Haltevorrichtung (23) zum Aufnehmen der Ventilvorrichtung (15) und eine Andockvorrichtung (25), die so ausgebildet ist, dass über sie die Sterilisierung durchgeführt wird, aufweist.

13. Anlage nach Anspruch 12, wobei die Andockvorrichtung (25) so ausgebildet ist, dass bei geöffnetem Ventil, die Haltevorrichtung (23), die Ventilvorrichtung (15) und die Andockvorrichtung (25) dichtend miteinander verbunden sind.

14. Anlage nach Anspruch 12 oder 13, wobei die Haltevorrichtung (23) ein Mittel zur Temperaturmessung (35) aufweist.

15. Anlage nach einem der Ansprüche 12 bis 14, wobei die Andockstation (25) eine Dampfzuführeinheit (53) zum Sterilisieren und eine Druckluftzuführeinheit (57) zur Überprüfung der dichten Anordnung der Haltevorrichtung (23), der Ventilvorrichtung (15) und der Andockvorrichtung (25) aufweist.

16. Anlage nach einem der Ansprüche 12 bis 15, wobei die zweite Sterilisationsstation (65) die Andockstation (25) der ersten Sterilisationsstation (21) umfasst.

17. Anlage nach Anspruch 16, wobei die Andockstation (25) so ausgebildet ist, dass die Andockvorrichtung (25) mit der Ventilvorrichtung (15) am Auslaufstutzen (9) anbringbar ist.

18. Anlage nach Anspruch 16 oder 17, wobei die Andockstation (25) eine Wasserstoffperoxidzuführeinheit (63) aufweist.

19. Anlage nach einem der Ansprüche 12 bis 18, mit einer Befüllstation zum Befüllen des Inliners (5), die eine dritte Sterilisationsstation (69) zum Sterilisieren produktführender Bereiche der Befüllstation des Anschlussbereichs umfasst.
